# EUROPEAN PATENT APPLICATION

(11) **EP 1 287 815 A1**
(43) Date of publication of application: **05.03.2003**
(21) Application number: 01203311.4
(22) Date of filing: 31.08.2001
(51) Int. Cl.: A61K 7/48

(54) **Use of a sphingoid base for inhibiting ceramidase activity**

(71) Applicant: Cosmoferm B.V., 2600 MA Delft (NL)
(72) Inventor: Nieuwenhuizen, Willem, Ferdinand, 3981 AM Bunnik (NL); Egmond, Maarten, Robert, 3511 GW Utrecht (NL); van der Wilden, Wim, 3054 CA Rotterdam (NL); Oudenaarde, Inge, 2907 PA Capelle a/d Ijssel (NL)
(74) Representative: Luys, Marie-José A.H.

(57) **Abstract**

Use of an active compound selected from the group of a sphingoid base, a derivative of a sphingoid base, a salt of a sphingoid base, a ceramide or a mixture of two or more of these compounds for manufacturing a composition for topical application for inhibiting ceramidase activity in and on the skin.

## Description

### Background of the invention.

Lipid lamellar structures in the intercellular spaces of the stratum corneum are considered responsible for the permeability function of the stratum corneum, in limiting loss of body fluids, in particular water, through the skin and limiting penetration of foreign external compounds through the skin (Elias, P.M., J. Invest. Derm. 1983, 80, 44-49). Ceramides are assumed to be the main lipid components within the intercellular lipid membranes and to be essential components in building and maintaining the barrier function of the stratum corneum. Other lipid components contributing to this barrier function include free fatty acids (mainly long chain free fatty acids), cholesterol, cholesteryl esters and cholesteryl sulphate.

The stratum corneum is built up of corneocytes that are received in a matrix of stacks of intercellular lipid membrane layers. Ceramides, as components of the lipid barrier, are thought to be responsible for holding the corneocytes together, similar to the model of mortar located between the bricks in a wall. Synthesis of ceramides takes place in the lower part of the stratum corneum. At the upper part of the stratum corneum, the ceramides are subjected to the action of ceramidases, which catalyse the hydrolysis of the ceramide N-acyl bond to generate a free fatty acid and a sphingoid base. In normal skin, equilibrium exists between ceramide synthesis and ceramide breakdown. Besides ceramidases, proteases play a role in the process on the surface of the skin to let the corneocyts squame off in a controlled way to remove old cells from the skin.

Various factors may adversely effect the ceramide content and thus the barrier function of the stratum corneum, such as for example exposure of the skin to detergents or solvents, ageing of the skin or exposure to UV-light. A damaged barrier function will result in a sensitive skin that for example looks red, dry and/or scaly, and/or result in skin diseases, like (atopic) dermatitis, psoriasis, xerosis or ichthyiosis.

It has been observed that the skin is populated with a wide variety of micro-organisms, including bacteria, yeasts and fungi. These micro-organisms secrete a wide variety of enzymes to the skin, including ceramidases. In this respect *Pseudomonas* has been found to be responsible for the release of an alkaline ceramidase on the stratum corneum. Ceramidases originating from *Pseudomonas* have been isolated from patients with atopic dermatitis (Nozomu Okino, J. Biol. Chem., 1999, Vol. 274, 51, pp 36616-36622).

The inventor has now analysed phenomena underlying the problem of an impaired permeability barrier function of the skin. It appeared to him that an impaired barrier function may be associated with a decreased ceramide content of the skin following increased ceramidase catalysed hydrolysis of the ceramide N-acyl bond, this ceramidase activity being responsible for distorting the equilibrium between ceramide synthesis and ceramide decomposition. There is thus a need to a composition, which is capable of inhibiting the activity of the ceramidases responsible for the unwanted hydrolysis of ceramides.

### Description of the prior art.

From EP-A-695.167 a composition for topical application is known, which is thought to be capable of increasing the ceramide content in the stratum corneum by stimulating ceramide synthesis in the epidermis. The composition of EP-A-695.167 contains one or more ceramide synthesis pathway intermediates or precursors thereof. Topical application of these compounds is supposed to promote the ceramide synthesis rate-limiting step in which serine and palmytoyl-CoA are converted to 3-ketosphinganine. Disclosed ceramide pathway intermediates or precursors thereof include N-acyl or O-acyl derivatives of sphinganine, sphingosine or phytosphingosine or mixtures thereof, for example tetra-acetylphytosphingosine. The composition may further comprise ceramide pathway adjuncts, for example saturated or unsaturated α-, β-, or ω-hydroxy fatty acids, cholesterol, cholesterol pathway intermediates or precursors thereof, ceramides etc.

### Description of the invention.

It is the aim of the present invention to provide a composition which is capable of affecting the activity of ceramidases responsible for the unwanted hydrolysis of ceramides.

This is achieved in the present invention by topical application of a composition with the technical features of the characterising part of the first claim.

According to the present invention, an active compound selected from the group of sphingoid bases, a derivative of a sphingoid base, a salt of a sphingoid base or a mixture of two or more of these compounds is used for manufacturing a composition for topical application for inhibiting ceramidase activity in and/or on the skin.

It has surprisingly been found that topical application of the composition of this invention has the effect that the activity of mammalian ceramidases present in the skin as well as the activity of microbial ceramides secreted by micro-organisms present on the skin, may be inhibited. The fact that the active compound of this invention is capable of inhibiting both mammalian and microbial ceramidase is surprising as it had been observed by the inventor that ceramidases of microbial origin do not respond to inhibition by well-known mammalian ceramidase inhibiting compounds. Examples of such mammalian ceramidase inhibiting compounds are D-erythro-MAPP ((1S,2R)-D-erythro-2-(N-myristoylamino)-1-phenyl-1-propanol) disclosed in US-A-5,830,916, or N-oleolyethanolamine (Sugita et al. Biochim Biophys Acta 398: 125 (1975)). The present finding that the composition of this invention is capable of inhibiting mammalian as well as microbial ceramidase may point to a still unknown similar active site and/or substrate-binding region present in mammalian and microbial ceramidase.

As there is a continuous synthesis of ceramides in the epidermis, inhibiting the ceramide decomposition will have the effect that a lacking amount of ceramide will be replenished and the over-all ceramide content of the stratum corneum may be maintained, restored and even increased. The present invention in fact provides a novel approach to repairing a damaged skin barrier. With the present invention the problem of the damaged barrier function is not solved by replenishing the lacking compound through topical application, but rather by treating and inhibiting the underlying effect causing damaging of the skin barrier, namely the ceramidase catalysed decomposition of ceramides. As the composition of this invention is capable of penetrating the skin, activity by microbial ceramidase secreted on top of the skin as well as ceramidase present in the stratum corneum may be inhibited.

Increasing the ceramide in the stratum corneum content has the effect that a locally distorted lipid barrier may be repaired and the lipid barrier may even be improved, which in the end will result in a thickening of the skin and an improvement of the over-all condition and quality of the skin. The increased ceramide content in the stratum corneum further allows preventing or repairing the occurrence of dry, itchy and/or rough skin, and reducing the occurrence of problems with skin sensitivity or skin diseases. Due to this thickening, ageing of the skin as well as the phenomena associated therewith, such as the occurrence of fine lines or wrinkles may be retarded. Also, inhibiting ceramide decomposition will assist in counteracting the occurrence of for example a dry or solvent affected skin, following impaired barrier function as a result of a decreased ceramide content.

It is known that environmental factors, like for example chemicals, detergents and UV may have a detrimental effect on the functioning of te skin barrier. As skin ceramidases act on the boundary between skin and the environment, these environmental factors may contribute to and even enhance the effect of ceramidases, thus increasing ceramide decomposition and thus deteriorating the barrier function of the skin. Further, the external factors as well as the consequential damaged skin barrier may involve a local increase of the number of micro-organisms on the skin. As a consequence, the amount of microbial ceramidase secreted on that part of the skin will be increased. It is likely that following an overcolonisation of micro-organisms on parts of the skin, the ceramidase concentration is locally increased and involve a locally distorted balance between ceramide synthesis and breakdown. The increased ceramide hydrolysis may lead to a ceramide level in the stratum corneum, which is insufficient to maintain the barrier function at the required level.

It is known from EP-A-814075 that sphingoid bases are able to inhibit topical growth of micro-organisms on the skin. As a consequence, topical application of the composition of the invention will not only have the effect that ceramidase activity is inhibited, but also that the growth of micro-organisms is counteracted. The decreased population by micro-organisms in turn will have the effect that the amount of ceramidase of microbial origin secreted on the skin will be minimised. Topical application of the composition of this invention thus produces a dual effect in that not only the concentration of the barrier lipids in the stratum corneum may be increased by inhibiting their decomposition by ceramidases, but also in that the overcolonisation of microbial flora will be counteracted by inhibiting the growth of the micro-organisms, thereby decreasing the amount of secreted ceramidases.

Benefits perceived by the consumer may be seen from improved skin barrier function involving fewer problems of skin sensitivity, skin diseases (such as atopic or diaper dermatitis, psoriasis, or ichthyiosis), photodamaged skin, loss of elasticity and flexibility, together with an improved skin condition, removal of age spots, keratoses, wrinkles, fine lines, coarse, rough and dry skin.

The observation that sphingoid bases, salts thereof or derivatives thereof are capable of inhibiting ceramidase activity has initiated a novel method of selectively treating increased ceramidase activity of mammalian and microbial origin in and on the skin, thereby maintaining, repairing or even enhancing the barrier function of the skin.

Mao C. et al (J. Biol. Chem., 2001; 276 (28): 26577-88) reported that expression of a human alkaline ceramidase (aPHC) in a yeast mutant strain caused an increased hydrolysis of phytoceramide in yeast cells. The human alkaline ceramidase has phytoceramidase activity both in vitro and in cells. Although it has been disclosed that human alkaline ceramidase is inhibited by sphingosine in vitro and in cells, it has nowhere been reported or taught that topical application of sphingosine results in an inhibition of both mammalian and microbial ceramidase. Nor has it been disclosed or taught that the ceramide content of the stratum corneum and the barrier function of the skin may be maintained, repaired or improved as a result of ceramidase-inhibition.

### Preferred embodiments of the invention.

The inhibitory effect has been found to be caused by an active compound selected from the group of sphingoid bases or derivatives or salts thereof or a mixture of two or more of these compounds. The sphingoid bases are preferably selected from the group comprising phytosphingosine, sphinganine, sphingosine, and 6-hydroxysphingosine. The active compound is preferably a D-enantiomer as this is the stereochemical configuration of the compound present in the mammalian body.

The composition of this invention preferably has a pH within the range of 4-9, preferably between 5-7, or between 4 and 5, between 7 and 9 or between 8 and 9, as these are the pH-ranges in which various types of ceramidases may be active.

The composition of this invention further preferably contains a compound capable of complexating divalent cations. The capturing of these divalent cations which are essential co-factors for the function of ceramidase prevents ceramidases from proper functioning. These divalent cations may originate from various sources and may for example be present on the skin, or be deposited on the skin from external sources. The simultaneous application of the active compound of this invention and the complexating agent has been found to produce a synergistic effect, according to which an improved inhibition of microbial ceramidase activity may be achieved.

The nature of the cation complexating compound is not critical to the invention and may be selected from the complexating agents generally known to the man skilled in the art. As the composition of this invention is intended for topical application, the complexating compound will preferably be selected such that it is a cosmetically or pharmaceutically acceptable ingredient, for example EDTA or citric acid.

The composition of this invention preferably comprises a protease inhibitor as it has a beneficial effect to the desquamation process of the skin. As they interfere in controlling the squaming off of corneocytes from the skin, to remove old cells from the skins, proteases also play an important role in maintaining the barrier function of the skin. The protease inhibitor will in general be present in a concentration of between 0.001-10 wt. %, preferably 0.01-5 wt. %.

The composition may further contain a cosmetically acceptable vehicle, any additional compound capable of supplementing the effect of the active compound as well as the usual adjuvants.

The present invention further relates to the use of the above-described composition for manufacturing of a cosmetic agent or a medicament for inhibiting ceramidases of mammalian and/or microbial origin.

Also pets and/or farm animals may benefit from topical application as they also suffer from itchy, dry, and/or scaly skin and/or skin diseases such as dermatitis.

### THE ACTIVE COMPOUND.

The active compound contained in the composition of this invention includes sphingoid bases, a derivative of a sphingoid base, a salt of a sphingoid base, or a mixture of two or more of these compounds.

Examples of suitable sphingoid bases include phytosphingosine, sphinganine, sphingosine and 6-hydroxysphingosine. The free sphingoid base present in the composition according to the invention has a general structure according to Formula 1: 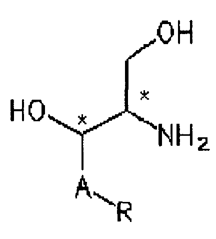 in which :
A is CH₂-CH₂-*(R)*, CH=CH-*(R)* or C(H)OH-CH₂-*(R)* or -CH=CH-CHOH-*(R)*
R is a straight chain or branched alkyl group having 10 to 22 carbon atoms which may optionally contain one or more double bonds and/or may optionally be substituted with one or more hydroxyl groups. R is preferably a straight chain alkyl group having 12 to 18 carbon atoms, more preferably a straight chain alkyl group having 13 carbon atoms. Both asymmetric carbon atoms may either take the D or L configuration.

The sphingoid bases used in the composition of this invention may be obtained from any suitable source, e.g. from a natural source or from a chemical synthesis process. It is desirable to apply a production process with which the sphingoid base may be obtained in the correct stereochemical configuration, in sufficient amounts at a commercially feasible price. Purification of animal and/or plant tissue extracts usually yields small amounts of sphingoid bases, making their isolation costly. Moreover, animal sources are believed to be unsafe due to the presence of viruses and other infectious agents, such as the agent causing BSE (mad cow's disease). Therefore, preferably use is made of a sphingoid bases obtained from a microbial fermentation process, using Pichia ciferrii as a yeast (US 6,204,006). Phytosphingosine may for example be obtained by a chemical or enzymatic catalysed deacetylation of Pichia ciferrii-derived tetraacetyl-phytosphingosine (TAPS). Phytosphingosine resulting from the alkaline TAPS hydrolysis may be purified by any method known to a person skilled in the art. Yeast-derived phytosphingosine is human skin-identical, as it is reported to have the same stereochemical configuration as mammalian phytosphingosine, i.e. the D-erythro configuration.

Examples of salts of sphingoid bases suitable for use with the present invention include those disclosed in WO 00/53568. The anion of the sphingoid base salt is preferably derived from an acid, which when mixed with the sphingoid base and a suitable solvent produces a salt with an improved solubility in aqueous medium, and has an efficacy in topical application. The acid may be a hydrophilic organic acid such as an α-hydroxy alkanoic acid, a β-hydroxy alkanoic acid, an α,β-hydroxy alkanoic acid, an alkanedioic acid or a mineral acid. Suitable organic acids include lactic acid, glycolic acid, malic acid, pyruvic acid, succinic acid, fumaric acid, citric acid, ascorbic acid, gluconic acid and/or pyroglutamic acid (pyrrolidone carboxylic acid), lipophilic organic acids. Preferred examples of mineral acids include hydrochloric acid, nitric acid and/or phosphoric acid.

Examples of derivatives of sphingoid bases include compounds corresponding to the formula 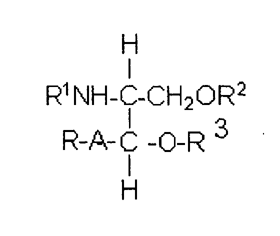 In which
A is CH₂-CH₂-*(R)*, CH=CH-*(R)* or C(H)OR⁴-CH₂-*(R)* or -CH=CH-CHOR⁴-*(R)*
R is as defined above,
R¹, R², R³, R⁴ are each individually represented by H-, a straight or a branched alkyl chain having between 1 and 30 carbon atoms, CH₃(CH₂)ₙ(CHOH)ₘ-, CH₃(CH₂)ₙ(CHOH)ₘC-, CH₃(CH₂)ₙC-, a B-O-D-C residue, 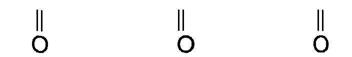 in which D is a C15-35 straight chain alkyl group which may optionally contain one or two double bonds, and B is a C12-20 straight chain acyl group which may optionally contain one or two double bond, for example lineoyl, stearoyl, oleoyl, phosphorylated, sulphated, glycosylated, benzoyl, -CO-C₅H₄OH (salicylic acid), retinoic acid, derivatives thereof, a 2-α-hydroxy acid residue having 3-40 carbon atoms for example lactic acid, glycolic acid, methyllactic acid, alpha hydroxycaprylic acid, alpha-hydroxylauric acid, or derivatives of these compounds,
where n is 0 or an integer between 1-30, preferably between 1-22, m is 0 or an integer between 1 and 5.

Examples of preferred sphingoid base derivatives for use with the present invention include retinoylamide derivatives of sphingoid bases disclosed in EP-B-789.686 and the lactic acid, and salicylic acid derivatives disclosed in EP-A-966.431. The more lipophilic character of the retinoylamide derivatives enhances penetration into the skin as compared to an unmodified retinoid.

Other preferred derivatives of sphingoid bases include their respective N-acyl, O-acyl and N-alkyl derivatives, containing a C1-C24 acyl or alkyl group. Particularly preferred sphingoid base derivatives include tetraacetyl-, triacetyl-, diacetyl phytosphingosine (TAPS), N-monoacetyl phytosphingosine (MAPS), triacety-, diacetyl and N-monoacetyl sphingosine, triacetyl- diacetyl and N-monoacetyl sphinganine. The N-atom may be dialkylated.

Suitable ceramides for use as an active component with the present invention are preferably selected from the group of ceramide 1, 1A, 2, 3, 3A, 3B, 4, 5, 6, 7 and 8. More specifically, the ceramides used with the present invention comprise a sphingoid base backbone selected from the group of sphingosine, 6-hydroxy sphingosine, phytosphingosine and sphinganine. The sphingoid base backbone may be acylated with an acyl or an acyloxyacyl group, which may have a variable chain length, be branched or not, contain one ore more double bonds, contain one or more hydroxyl groups. Preferred ceramides may be selected from the group of N-tetracosanoyl phytosphingosine, N-stearoyl phytosphingosine, N-oleoyl phytosphingosine, N-linoleoyl-phytosphingosine, N-(2-hydroxytetracosanoyl) phytosphingosine, N-(2-hydroxyoctadecanoyl) phytosphingosine, N-(27-stearoyloxyheptacosanoyl) phytosphingosine, N-(27-oleoyloxyheptacosanoyl) phytosphingosine, N-(27-linoleoyloxyheptacosanoyl) phytosphingosine, N-(23-stearoyloxytricosanoyl) phytosphingosine.

As the active component use may also be made of a sphingolipid, for example a glycoceramide. With respect to glycoceramides, two groups of these compounds are typically distinguished, i.e. cerebrosides and gangliosides. A cerebroside is understood to be a glycoceramide wherein a monosaccharide, mostly glucose or galactose, is attached to the oxygen of the -CH2OH group. In gangliosides, oligosaccharides frequently including sialic acid, are attached to the same.

When incorporated into a formulation, the concentration of the active compound will preferably be varied from 0,0001 - 15 wt. % to allow achieving the desired inhibitory effect. More preferred ranges include between 0.001 - 10 wt %, 0.01- 5 wt. % and 0.01-2.5 wt. % as these doses allow achieving the required inhibitory effect, while minimising the cost of the composition.

### THE COSMETICALLY ACCEPTABLE VEHICLE.

The composition of this invention also comprises an amount of a cosmetically acceptable vehicle, which may be solid, semi-solid, viscous, liquid to enable topical application of the composition. The man skilled in the art will be able of selecting the appropriate vehicle depending on the intended application and way of administration.

Vehicles other than water can include liquid or solid emollients, solvents, humectants, thickeners, powders, surfactants, which are also sometimes designated as emulsifiers, solubilizers, propellants and other active ingredients.

Suitable emollients include (fatty acid) esters, fatty acids, (fatty) alcohols, polyols, (natural) waxes, natural oils, silicone oils, both volatile and non-volatile and hydrocarbons such as mineral oil, petroleum jelly, vaseline, squalens and (iso)paraffin.

### ADDITIONAL INGREDIENTS.

The nature of the complexating compound for capturing divalent cations is not critical to the invention and may be selected by the man skilled in the art from the wide variety of complexating agents known to him. Suitable examples include EDTA, citric acid, or any cosmetically or pharmaceutically acceptable organic acid containing two or three or even more carboxylic acid groups.

The cation complexating compound is preferably present in an amount 0,0001 - 25 wt. % to allow achieving the desired inhibitory effect. However, depending on the molecular weight of the complexating compound the concentration may also be varied from 0.005 - 5 % or 0.01-1 wt. %.

The composition of the invention optionally may comprise one or more additional skin lipid compounds, such as cholesterol, cholesterol esters like cholesteryl sulphate, free fatty acids like palmitic, stearic, behenic, oleic and/or linoleic acid and/or other sphingolipids.

Other additional ingredients include those compounds, which as such have beneficial effect to the skin. For instance, yeast β-glucan may be applied in the composition according to the invention to reduce UV-induced erythema.

Suitable propellants for use with the present invention include propane, butane, isobutane, dimethyl ether, chlorofluoroalkanes, carbon dioxide, nitrous oxide.

Solvents include ethyl alcohol, methylene chloride, isopropanol, ethyl ethers such as ethoxyethanol and butoxyethanol, acetone, tetrahydrofuran, dimethylformamide, DMSO, propylene glycol, butylene glycol.

Humectants include proteins and protein hydrolysates, amino acids, sorbitol, glycerin, sorbitol, glycols preferably PEG 200-4000 and other polyols.

Thickeners include cross linked polyacrylates, silicone gums and polysaccharide gums such as xanthan, carrageenan, gelatin, pectin and locust beans gum, hyaluronic acid and carboxylic group-containing polymers.

Powders include chalk, talc, starch, kaolin, clays, silicates, carboxyvinyl polymers.

Other active ingredients include:
- anti-oxidants like butyl hydroxy toluene, hydroquinone, tocopherols, gallates;
- preservatives like para-hydroxy benzoate esters, sorbic acid, , quaterniums, benzoic acid, imidazolidinyl urea, (benzyl)alcohol;
- sunscreens, blocking UV light, like PABA's, and cinnamate

The combination of the said components can account for 5 to 99% of the composition.

### USE OF THE COMPOSITION.

The composition of this invention is intended for topical application to the skin, which includes human as well as animal skin, the human scalp and animal fur. Topical application is understood to comprise cosmetic and/or dermatological application on the skin, on hair and on the epithelial linings of mouth, nose, eye, urogenital tract, and the like.

The composition may be used as such, or be incorporated in a further formulation for topical application. The composition of this invention is intended for use as a cosmetic agent for preventing, reducing, or treating the signs of ageing of the skin, thin skin, fine lines and/or wrinkles, sensitive skin, induced coarse, dry and/or scaly, damaged skin, repairing skin barrier function where this is imparted by increased ceramidase activity and in general for improving the quality of the skin. The composition of this invention is also intended for use as a pharmaceutical composition for treating skin deseases such as for example dermatitis, psoriasis, ichtyosis.

If microbial ceramidase activity is negligeble, the composition of this invention may be used for exclusively controlling mammalian ceramidase activity.

Topical compositions according to the invention comprising the active ingredient may be applied in the form of a cream, lotion, gel, emulsion, paste, ointment, aerosol, spray, spot-on, shampoo, plaster, dressing, and other forms of application known to the man skilled in the art.

Examples of topically occurring undesirable and/or abnormal conditions in which topical compositions comprising the active ingredient are advantageously applied are: psoriasis, acne, dandruff, mouth and/or lip infections, mycoses, various other skin-infectious diseases or vaginal infections, wound-healing, normalisation of skin flora.

Through topical application of the composition of this invention a synergistic effect may be achieved. According to this effect a maintaining, restoring, repair or even an improvement of the ceramide content of the skin may be achieved, by treating the phenomenon causing ceramide decomposition. On the one hand, topical application of the composition of this invention allows inhibiting the activity of ceramidases naturally present in the skin, as well as the activity of ceramidases secreted by micro-organisms present on the skin. Besides this, topical application of the composition of this invention also allows controlling the amount micro-organisms present on the skin by inhibiting their growth. In that way the amount of microbial ceramidase released to the skin may be controlled. All of the above mentioned effects contribute to curing repairing, enhancing or maintaining the barrier function of the skin.

The present invention also relates to a method for inhibiting topically occurring ceramidase activity, comprising topical application of a topical composition comprising an active compound selected from the group of a sphingoid base, a derivative of a sphingoid base, a salt of a sphingoid base or a mixture of two or more of these compounds.

The invention is further elucidated in the following examples and comparative examples.

### Examples.

The effect of various active compounds on the enzymatic activity of recombinant ceramidase from *Pseudomonas aeruginosa* PA01 was tested.

The assays were performed at 25°C, with magnetic stirring, using Photo Technology International fluorimeter equipped with a LPS 220 Lamp Power Supply, a 810 Photomultiplier Detector System, a MD 5020 Motor Driver, and a Dell Dimensions M166a computer with FELIX software. Excitation was done at 346 nm (± 2 nm) and the emission was measured at 378 nm (± 2 nm). The assay buffer consist of 50 mM Tris/HCl, 4 mM CaCl₂, 0.1% w/v Triton X-100, pH 8.5.

The active compounds were dissolved in DMF to a concentration of 4 mM. Of these solutions, 250 µliter was added to 50 milliliter assay buffer under vortex, yielding 20 µM lipid solutions.

To 1 mL of the lipid solution in a cuvet, 5 µL of quenched fluorescent ceramide analog as substrate was added (end concentration 2 µM) and the system was allowed to equilibrate for 5 minutes. Then 5 µL of recombinant ceramidase was added (end concentration 4.3 nM). The reaction was allowed to proceed for 20 minutes. The slope at 378 nm was used to determine the reaction rate.

Control experiments were done in the absence of sphingolipids, with corresponding amounts of DMF (0.5% v/v for the 20 µM samples). The reaction rate of the controls was set at 100%. All assays were done in duplo, and the average values were used to calculate the values given in the table below. The percentages in the table are given as % of inhibition (control value - reaction rate of the sample).

### Comparative example I.

The potential of several known human ceramidase inhibiting compounds of inhibiting *Pseudomonas aeruginosa* PA01 ceramidase was tested at various concentrations, in the assay buffer containing 20 µM of substrate.

The microbial inhibiting potential of the following compounds was determined: D-erytro-MAPP, L-erythro-MAPP (the inactive enantiomer of D-erytro-MAPP), N-oleoylethanolamine, L(-)-norephenidrin, D(+)-norephenidrin. None of these compounds was found capable of inhibiting the *Pseudomonas aeruginosa* ceramidase.

### Example 1-9.

The potential of several ceramide and sphingoid bases of inhibiting *Pseudomonas aeruginosa* PA01 ceramidase was tested at a pH of 8.5, using respectively 2µM and 20 µM of ceramidase substrate, 1.6 mM Triton X-100 and 4 mM of CaCl₂. The ability of the active compounds of inhibiting ceramidase activity are given in % of inhibition (control value - reaction rate of the sample).

As can be seen from the table, D-erythro sphingosine (example 8) shows the highest ceramidase inhibition ability, followed by the mixture of D and L sphinganine (example 7). Based on the observation that the inhibiting effect produced by L- erythro-sphingosine is lower as compared to the D-enantiomer, it is expected that the inhibiting effect of D-sphinganine will be better than the inhibiting effect of the racemic mixture.

Phytosphingosine.HCI was found to have a better ceramidase inhibiting effect than phytosphingosine (example 2 and 3).

From example 8 and 9 it appears that the ceramidase has a stronger chiral preference for the binding of D-erythro sphingosine as compared to the L-enantiomer, as is reflected in the different inhibiting power of both enantiomers.

| | % of inhibition | |
|---|---|---|
| Concentration of active compound | 20 µM | 2 µM |
| Active compound | | |
| 1. Ceramide VI | 11 | |
| 2. Phytosphingosine | 60 | 7.5 |
| 3. Phytosphingosine.HCI | 67 | 15 |
| 4. N-acetyl-phytosphingosine | 9 | |
| 5. MAPS | 35 | |
| 6. TAPS | 22 | |
| 7. D/L sphinganine | 83 | |
| 8. D-erythro-sphingosine | 97 | |
| 9. L- erythro-sphingosine | 20 | |

## Claims

1. Use of an active compound selected from the group of a sphingoid base, a derivative of a sphingoid base, a salt of a sphingoid base, a ceramide or a mixture of two or more of these compounds for manufacturing a composition for topical application for inhibiting ceramidase activity in and on the skin.

2. Use as claimed in claim 1, **characterised in that** the sphingoid base is selected from the group of sphingosine, 6-hydroxysphingosine, phytosphingosine, sphinganine or a mixture of two or more of these compounds.

3. Use as claimed in claim 1 or 2, **characterised in that** the sphingoid base derivative is selected from the group of a N-salicyloyl-sphingoid base, N-lactyloyl-sphingoid base, N-retinoyl-sphingoid base, N-acetyl sphingid base, diacetyl-, triacetyl- or tetraacetyl sphingoid base, preferably from the group of di- or triacectyl phytosphingosine or di- or triacetyl sphinganine or a mixture of two or more of these compounds.

4. Use as claimed in any one of claims 1-3, **characterised in that** the sphingoid base salt is selected from the group of sphinhoid base salts of ascorbic acid, citric acid, fumaric acid, gluconic acid, glycolic acid, hydrochloric acid, lactic acid, malic acid, pyroglutamic acid or a mixture of two or more of these compounds.

5. Use as claimed in any one of claims 1-4, **characterised in that** the ceramide is selected from the group of ceramide 1, 1A, 2, 3, 3A, 3B, 4, 5, 6, 7, 8 and glycoceramides.

6. Use as claimed in any one of claims 1-5, **characterised in that** as the active compound use is made of a compound which has a D-erythro stereochemical configuration.

7. Use as claimed in any one of claims 1-6, **characterised in that** the composition further contains a compound capable of complexating divalent cations.

8. Use as claimed in any one of claims 1-7, **characterised in that** the complexating compound is selected from the group of di- and tri-carboxylic organic acids, fatty acids, EDTA, citric acid.

9. Use as claimed in any one of claims 1-8, **characterised in that** the composition has a pH of between 4 and 9, preferably between 5 and 7.

10. Use as claimed in any one of claims 1-9, **characterised in that** the active compound is present in a concentration of between 0.0001-15 wt. %, preferably between 0.001 - 10 wt %, more preferably between 0.01-2.5 wt. %

11. Use as claimed in any one of claims 1-10, **characterised in that** the composition contains a protease inhibiting agent.

12. Use as claimed in any one of claims 1-11, for inhibiting ceramidase of mammalian and/or microbial origin.

13. Use of the composition of any one of claims 1-12 for manufacturing a composition for maintaining, repairing, restoring or enhancing a permeability barrier function of the skin which has been disturbed by ceramidase activity.

14. Use as claimed in any one of claims 1-13 for preventing, reducing or treating signs of ageing of the skin, thin skin, fine lines and/or wrinkles, sensitive skin, dry and/or scaly skin.

15. Use as claimed in any one of claims 1-14 for manufacturing a composition for inhibiting ceramidase activity in and on mammalian skin and/or scalp.

16. Use as claimed in any one of claims 1-13 for manufacturing a composition for inhibiting ceramidase activity in and on animal skin and fur.

17. Use as claimed in any one of claims 1-16 for the manufacturing of a cosmetic composition.

18. Use as claimed in any one of claims 1-16, for the manufacturing of a medicament, preferably a medicament for treating skin diseases such as dermatitis, psoriasis, ichthyosis.

19. A method for inhibiting topically occurring ceramidase activity, comprising topical application of a composition comprising an active compound selected from the group of a sphingoid base, a derivative of a sphingoid base, a salt of a sphingoid base or a ceramide, or a mixture of two or more of these compounds.

20. The method of claim 19, **characterised in that** the sphingoid base is selected from the group of sphingosine, 6-hydroxysphingosine, phytosphingosine, sphinganine or a mixture of two or more of these compounds, the sphingoid base derivative is selected from the group of a N-salicyloyl-sphingoid base, N-lactyloyl-sphingoid base, N-retinoyl-sphingoid base, diacetyl-, triacetyl- or tetraacetyl sphingoid base, preferably from the group of di- or triacectyl phytosphingosine or di- or triacetyl sphinganine or a mixture of two or more of these compounds, the sphingoid base salt is selected from the group of sphinhoid base salts of ascorbic acid, citric acid, fumaric acid, gluconic acid, glycolic acid, hydrochloric acid, lactic acid, malic acid, pyroglutamic acid or a mixture of two or more of these compounds, and the ceramide is selected from the group of ceramide 1, 1A, 2, 3, 3A, 3B, 4, 5, 6, 7, 8 and glycoceramides.
